# EUROPEAN PATENT SPECIFICATION

(11) **EP 3 010 521 B1**
(45) Date of publication and mention of the grant of the patent: **10.01.2018**
(21) Application number: 14737031.6
(22) Date of filing: 16.06.2014
(51) Int. Cl.: A61K 38/00, A61K 31/702, A61K 35/74, A23L 33/17, A61P 31/00

(54) **SYNBIOTIC COMPOSITION FOR TREATMENT OF INFECTIONS IN ALLERGIC PATIENTS**
SYMBIOTISCHE ZUSAMMENSETZUNG ZUR BEHANDLUNG VON INFEKTIONEN BEI ALLERGISCHEN PATIENTEN
COMPOSITION SYMBIOTIQUE POUR LE TRAITEMENT D'INFECTIONS CHEZ DES PATIENTS ALLERGIQUES

(30) Priority: 14.06.2013 WO PCT/NL2013/050423
(43) Date of publication of application: 27.04.2016
(73) Proprietor: N.V. Nutricia, 2712 HM Zoetermeer (NL)
(72) Inventor: HARTHOORN, Leunis Forrinus, 3584 CT Utrecht (NL)
(74) Representative: Nederlandsch Octrooibureau
(86) International application number: PCT/NL2014/050392
(87) International publication number: WO 2014/200351

(56) References cited:
- EP-A1- 1 714 660
- EP-A2- 2 033 529
- WO-A1-2011/149336
- US-A1- 2011 097 437
- US-A1- 2013 089 572
- US-B2- 8 425 955

## Description

### FIELD OF THE INVENTION

The present invention relates to nutritional compositions comprising synbiotics for use in the treatment or prevention of infections in allergic patients.

### BACKGROUND OF THE INVENTION

Synbiotic combinations of probiotic lactic acid bacteria and prebiotic indigestible fibers have been tested in models for inflammation and in human studies. Although reductions in inflammatory responses have been shown in several treatment protocols, results are conflicting and not consistent depending on the model or patient group used. Allergy has long been related to improved hygiene in the developed world. Based on this hygiene hypothesis a large number of studies have been done where it was tried to treat allergy, or improve the allergic symptoms, e.g. atopic dermatitis. The allergic patients were treated with probiotic bacteria or with dietary fibers or both, but the results allergy on prevention were inconsistent.

For example in Allergy (2011) 66:170-177 van der Aa et al. reported effects on asthma symptoms but the number of respiratory infections (lower and upper) during the intervention period did not differ between the synbiotic and the placebo group. The treatment product used in this study was an infant formula with galactooligosaccharides and inulin as prebiotics and B.breve as probiotic.

Currently, probiotics or prebiotics are not commonly used for treating infections in allergic patients.

Kukkonen et al., J Allergy Clin Immunol (2007) 119: 192-198 described a study using synbiotics that consisted of 4 probiotic strains and prebiotic galactooligosaccharides. The synbiotics were given in a double-blinded manner to pregnant mothers and to their healthy infants from birth to the age of 6 months. It is not reported if the infants were allergic. Kukkonen finds indications for an inverse association between modification of the indigenous gut microbiota and the prevalence of eczema, especially when IgE associated. This preventive study did not use a nutritional composition with synbiotics but used capsules to be eaten by the mother or to be mixed with liquids for the infant. It is also not disclosed what the effect would be of the synbiotics when given to allergic infants.

WO 2010/033768 discloses compositions including infant formula comprising probiotics for reducing inflammation. The inflammation may be caused by allergy, chronic inflammatory disease, etc. An inflammation is an immune reaction that can result from an infection. Inflammation is in general an immune response of the body against a harmful stimulus such as pathogenic micro organisms, chemicals, damaged tissues. The treatment or prevention of infections is thus different from treating an inflammation and the document does not disclose the treatment or prevention of infections in allergic patients, but only the treatment of inflammation.

Böhme et al. in Acta Derm Venereol. (2002) 82(2):98-103 describe that during the first 2 years of life there is a significant association between atopic dermatitis and respiratory infections manifested in an increased rate of acute otitis media, pneumonia and use of antibiotics. It is known that these infections often exacerbate the allergic manifestations. There is thus a real need to limit the microbial infection rate in allergic patients.

EP 1714660 discloses compositions containing probiotic bacteria and uronic acid oligosaccharides that are suitable as infant nutrition and advantageously reduce the incidence of infection.

### SUMMARY OF THE INVENTION

The inventors have surprisingly found for the first time that a synbiotic, i.e. a combination of a probiotic lactic acid bacterium and an indigestible fiber significantly reduced the microbial infection rate in allergic patients when given in a hypoallergenic formula, see example. In addition a statistical significant decrease in antibiotic use was found in the treatment group receiving the synbiotic composition.

Advantageously the present synbiotic composition provides the treatment of the infection and not the inflammation that can result from an infection. A beneficial consequence is that the inflammation can be prevented and therefore there is no need any more for treating the inflammation in a later stage, for example by administering analgesia or COX enzyme inhibitors like ibuprofen.

### DETAILED DESCRIPTION OF THE INVENTION

The present invention is defined by the claims. The present invention thus concerns a composition comprising i) a protein source, ii) a prebiotic and iii) a probiotic according to claim 1 for use in the treatment or prevention of infection in an allergic subject. The present disclosure thus concerns a method for the treatment or prevention of infection in an allergic subject, said method comprising administering a composition comprising i) a protein source consisting essentially of free amino acids, ii) at least one soluble indigestible fiber selected from the group consisting of fructooligosaccharides, non-milk derived fucosyloligosaccharides and polydextrose, and iii) at least one lactic acid bacterium selected from the group consisting of *Bifidobacterium breve, Bifidobacterium longum, Bifidobacterium infantis, Bifidobacterium lactis* and *Lactobacillus rhamnosus,* to said allergic subject.

In other words the disclosure concerns the use of i) a protein source, ii) a prebiotic and iii) a probiotic for the manufacture of a nutritional composition for the treatment or prevention of infection in an allergic subject, wherein i) the protein source consists essentially of free amino acids, ii) the prebiotic comprises at least one soluble indigestible fiber selected from the group consisting of fructooligosaccharides, non-milk derived fucosyloligosaccharides and polydextrose, and iii) the probiotic comprises at least one lactic acid bacterium selected from the group consisting of *Bifidobacterium breve, Bifidobacterium longum, Bifidobacterium infantis, Bifidobacterium lactis* and *Lactobacillus rhamnosus.*

The disclosure can also be worded as a composition comprising i) a protein source consisting essentially of free amino acids, ii) at least one soluble indigestible fiber selected from the group consisting of fructooligosaccharides, non-milk derived fucosyloligosaccharides and polydextrose, and iii) at least one lactic acid bacterium selected from the group consisting of *Bifidobacterium breve, Bifidobacterium longum, Bifidobacterium infantis, Bifidobacterium lactis* and *Lactobacillus rhamnosus,* for use in the treatment or prevention of infection in an allergic subject.

### Lactic acid bacteria

The fecal flora of breast fed infants is dominated by bifidobacteria, due to the presence of oligosaccharides in human milk. These act as bifidogenic factors, stimulating the proliferation of these species in the infant gut. Bifidobacteria are among the earliest colonizers of the human gastrointestinal tract and their presence in large numbers in the intestines of breast-fed infants has been associated with improved health. Atopic infants have been shown to have an altered gut microflora with increased clostridia and decreased bifidobacteria. The bifidobacteria microflora of atopic infants has been shown to be more adult like with decreased strains of *B. bifidium* and *B. breve* and increased *B. adolescentis.*

The composition for use according to the present disclosure comprising the invention comprises at least one lactic acid bacterium selected from the group consisting of *Bifidobacterium breve, Bifidobacterium longum, Bifidobacterium infantis, Bifidobacterium lactis,* and *Lactobacillus rhamnosus.* Typically, these lactic acid bacteria are commercially available from producers of lactic acid bacteria, but they can also be directly isolated from faeces, identified, characterised and produced.

The composition for use according to the present invention comprises at least 1.0x10⁹ living lactic acid bacteria (colony forming units; CFU) per liter, preferably between 1.0x10⁹ and 1x10¹¹ CFU per liter. Preferably the composition for use according to the present invention comprises at least 1.0x10⁷ living lactic acid bacteria (colony forming units; CFU) per gram dry weight, preferably between 1.0x10 and 1x10⁹ CFU per gram dry weight.

It is important for the synbiotic effect of the lactic acid bacteria and the prebiotic fiber that the concentration of the two ingredients is well balanced. Therefore preferably the concentration of the lactic acid bacteria is at least 1.0x10⁸ CFU lactic acid bacteria per gram prebiotic fiber, even more preferably between 2.0x10⁸ and 2.0x10¹⁰ CFU lactic acid bacteria per gram prebiotic fiber, more preferably between 1.0x10⁹ and 1.0x10¹⁰ CFU lactic acid bacteria per gram prebiotic fiber, most preferably between 1.0x10⁹ and 5.0x10⁹ CFU lactic acid bacteria per gram prebiotic fiber.

In one instance according to the method or use according to the present invention, the composition is administered in an amount that provides at least 1.0x10⁷ CFU lactic acid bacteria per day, preferably in an amount that provides from at least 2.0x10⁷ to at most 2.0x10¹¹ CFU lactic acid bacteria per day, in an amount that provides from at least 4.0x10⁷ to at most 1.2x10¹¹ CFU lactic acid bacteria per day, even more preferably in an amount that provides from at least 1.0x10⁸ to at most 6.0x10¹⁰ CFU lactic acid bacteria per day.

*Lactobacillus rhamnosus,* in particular *Lactobacillus rhamnosus GG,* also referred to as *Lactobacillus GG* or LGG, is one of the best studied species in humans and is also found in high amounts in the gut of infants. In a preferred embodiment the at least one lactic acid bacterium is selected from the group consisting of *Bifidobacterium breve, Bifidobacterium longum, Bifidobacterium infantis, Bifidobacterium lactis* and *Lactobacillus rhamnosus GG.* LGG is commercially available and can be obtained from Valio Ltd.

Bifidobacterium is a genus of Gram-positive, non-motile, often branched anaerobic bacteria. Bifidobacteria are ubiquitous, endosymbiotic inhabitants of the gastrointestinal tract, vagina and mouth of mammals and other animals. Some bifidobacteria are used as probiotics. In a preferred embodiment, the lactic acid bacterium in the composition for use according to the present invention is *Bifidobacterium breve,* or in one embodiment consist of *Bifidobacterium breve.* According to a preferred embodiment, the composition for use according to the present invention comprises at least one *B. breve* selected from the group consisting of *B. breve* Bb-03 (Rhodia/Danisco), *B. breve* M-16V (Morinaga), *B. breve* R0070 (Institute Rosell, Lallemand), B. breve BR03 (Probiotical), B. breve BR92) (Cell Biotech), DSM 20091, LMG 11613, YIT4065, FERM BP-6223 and CNCM I-2219. Most preferably, the *B. breve* is selected from the group consisting of *B. breve* M-16V and *B. breve* CNCM I-2219, most preferably M-16V. *B. breve* I-2219 was published in WO 2004/093899 and was deposited at the Collection Nationale de Cultures de Microorganisms, Institute Pasteur, Paris, France on 31 May 1999 by Compagnie Gervais Danone. *B. breve* M-16V was deposited as BCCM/LMG23729 and is commercially available from Morinaga Milk Industry Co., Ltd.

Preferably the bacteria are alive, however, non-living lactic acid bacteria can also have beneficial effects on the immune system. Without being bound by theory it is hypothesized that dead lactic acid bacteria can be used in the treatment or prevention of infection in allergic patients. In a preferred instance at least part of the lactic acid bacteria present in the composition are dead or at least not capable to multiply.

### Indigestible fiber

Soluble indigestible fiber is a term known in the art and refers to non digestible carbohydrate that can be used by the probiotic bacteria as a source of energy (fermentation) in the intestinal tract. Most of the formation and proliferation of the probiotic bacteria will take place in the colon. Without being bound by theory the inventors believe that administering live probiotic bacteria enteraly results in a relatively high concentration of these microorganisms in the small intestines where the fermentation can start resulting in fermentation products that are beneficial for the stimulation of the immune system resulting in a lower infection rate.

Thus, a soluble indigestible fiber can be defined as a non-digestible carbohydrate that beneficially affects the host by selectively stimulating the growth and/or activity of one or a limited number of bacteria in the colon. Preferred soluble indigestible fibers in the composition for use according to the present invention are not milk derived. Preferred soluble indigestible fibers in the composition for use according to the present invention include fructooligosaccharides, polydextrose and non-milk derived fucosyloligosaccharides, such as fucosyllactoses, fucosylated lactosamine-lactoses, and the like, and sialylated oligosaccharides characterized by one or more residues of N-acetylneuraminic acid, such as 3'- and 6'-sialyllactose (SL) and sialyl-lacto-N-tetraose.

The term "oligosaccharide" as used in the present invention preferably refers to a saccharide with an average degree of polymerization (DP) of 2 to 100, more preferably an average DP of 2 to 60. It is understood that in the context of this invention an oligosaccharide with a DP in a certain range may include a mixture of saccharides with different average DP's, for example, if an oligosaccharide with a DP of 2 to 100 is included in the present composition, this may include compositions which contain oligosaccharides with an average DP between 2 and 5, an average DP between 50 and 70 and an average DP between 7 and 60.

In one instance, the composition for use according to the present invention does not comprise uronic acid oligosaccharide, preferably the composition for use according to the present invention does not comprise uronic acid oligosaccharide with a degree of polymerization of 2 to 250. The term uronic acid oligosaccharide as used herein refers to an oligosaccharide wherein at least 50% of the residues are selected from the group consisting of guluronic acid, mannuronic acid, galacturonic acid and glucuronic acid.

In one instance the soluble indigestible fibers in the composition for use according to the present invention comprise polydextrose. Polydextrose is a soluble indigestible fiber favorably fermented by Bifidobacteria and Lactobacilli. It has the additional advantage of delivering only 1 kcal per gram of fiber, compared to 2 kcal per g for fructooligosaccharides. It is widely used and can be commercially obtained for example under the trade names LITESSE, STA-LITE, and TRIMCAL.

In a preferred instance the soluble indigestible fibers in the composition for use according to the present invention comprise fructooligosaccharides. The term "fructooligosaccharide" as used herein refers to a soluble indigestible fiber comprising a chain of at least 2 β-linked fructose units. A fructooligosaccharide can comprise a terminal glucose unit. In a preferred instance, the average degree of polymerisation of the fructooligosaccharides in the composition for use according to the present invention is in the range of 2 to 60, preferably the degree of polymerisation of the fructooligosaccharides is in the range from 2 to 60.

Preferably the soluble indigestible fibers in the composition for use according to the present invention is a combination of short chain fructooligosaccharides (scFOS) and long chain fructooligosaccharides (lcFOS). Long chain fructooligosaccharides is also referred to as inulin. Preferably the ratio scFOS : lcFOS is in the range of 95/5 to 10/90, even more preferably in the range of 95/5 to 40/60. In the context of this invention, scFOS has an average DP between 2 and 6. In the context of this invention lcFOS means any fructooligosaccharide composition with an average DP larger or equal to 7. A suitable source of scFOS is RAFTILOSE® (Orafti). RARTILINE® HP (Orafti) is a particularly preferred source of lcFOS and has an average DP > 20. Products commonly marketed as inulin comprise scFOS and lcFOS has in general an average DP larger than 7.

The composition for use according to the present invention preferably comprises more scFOS than lcFOS. Preferably the ratio scFOS : lcFOS is at least 1, preferably between 2 and 12, even more preferably between 3 and 10, most preferably the ratio scFOS : lcFOS is about 9. Both scFOS and lcFOS stimulate the growth of Bifidobacteria and Lactobacilli. It has been found that scFOS stimulates the growth already at the beginning of the colon, while the lcFOS stimulates the growth of the bacteria at the distal part of the colon.

The soluble indigestible fiber is preferably present in the composition for use according to the invention in an amount to provide a dose of 0.1-7 g/day more preferably 0.2 to 6 g/day, even more preferably 0.5 to 3 g/day. In one instance according to the method or use according to the present invention, the composition is administered in an amount that provides 0.1-7 g soluble indigestible fiber per day more preferably 0.2 to 6 g soluble indigestible fiber day, even more preferably 0.5 to 3 g soluble indigestible fiber day.

The soluble indigestible fiber is preferably present in a concentration of at least about 15 mg per gram dry weight of the composition, or at least 3 gram per liter composition. More preferably the concentration of soluble indigestible fiber in the composition for use according to the present invention is from 15 to 75 mg per g dry weight of the composition, and even more preferably from 35 to 60 mg per g dry weight of the composition.

Galactooligosaccharides (GOS) commonly used as prebiotic fiber in nutritional composition, including infant formula, is not suitable for the purpose of the present invention. GOS is derived from milk lactose, and is normally polluted with small amounts of milk protein. This milk protein, although present in small amounts, can still trigger immune reactions in the allergic patient. Thus in one embodiment, the composition for use according to the present invention does not comprise galactooligosaccharides.

### Protein source

Allergy patients normally have an overreacting immune response against protein allergens. In particular food allergy is caused by many food related proteins. Cow's milk proteins are the most common allergens in infancy, followed by chicken egg proteins. In order to be absolutely sure that no protein is present in the composition for use according to the invention, the protein source exclusively consists of free amino acids.

The present invention advantageously concerns the use of a composition wherein the protein source provides 7 to 20% of the total calories of the composition, preferably the protein source provides 8 to 17% of the total calories, even more preferably the protein source provides 9 to 15% of the total calories of the composition.

Alternatively, in the composition for use according to the present invention, the content of the protein source is between 10 and 20 wt% free amino acids based on dry weight of the total composition, preferably between 11 and 18 wt%, and even more preferably between 12 and 16 wt% free amino acids based on dry weight of the total composition. Thus the composition for use according to the present invention comprises as the sole protein source between 10 and 20 wt% free amino acids, preferably between 11 and 18 wt%, and even more preferably between 12 and 16 wt% free amino acids, based on dry weight of the total composition.

In one instance, the composition for the use according to the present invention is an infant formula. Therefore in one instance, the protein source comprises all essential amino acids. The optimal amino acid profile for infant formula is know in the art. A preferred instance of an amino acid composition is given in table 2.

### Fat

The composition for use according to the present invention preferably comprises fat. The term 'fat' as used in the present invention includes all fat sources commonly used in nutritional products and may comprise a source of triglycerides, diglycerides, monoglycerides or free fatty acids. In particular when the composition for use according to the invention is for the treatment of infants, the composition preferably comprises long-chain polyunsaturated fatty acids (LCPUFA). In a preferred instance the composition for use according to the present invention comprises eicosapentaenoic acid (EPA), arachidonic acid (ARA) or docosahexaenoic acid (DHA), preferably the composition comprises ARA or DHA or both, more preferably the composition comprises ARA and DHA. In a preferred instance the fat provides 30 to 50% of the total calories of the composition.

In a preferred instance according to the present invention the composition comprises at least 0.05 g ARA and/or at least 0.05 g DHA per liter composition, or even more preferably from at least 60 mg to at most 420 mg ARA per liter final composition and/or from at least 60 mg to at most 420 mg DHA per liter final composition or even more preferably from at least 80 mg to at most 240 mg ARA per liter final composition and/or from at least 80 mg to at most 240 mg DHA per liter final composition. In one instance the composition for use according to the present invention comprises at least 0.35 mg ARA per g dry weight of the composition and/or at least 0.35 mg DHA per g dry weight of the composition. Preferably the composition comprises from at least 0.4 mg to at most 10 mg ARA per g dry weight of the composition and/or from at least 0.4 mg to at most 10 mg DHA per g dry weight of the composition, preferably from at least 0.5 mg to at most 6 mg ARA per g dry weight of the composition and/or from at least 0.5 mg to at most 6 mg DHA per g dry weight of the composition, more preferably from at least 0.6 mg to at most 3 mg ARA per g dry weight of the composition and/or from at least 0.6 mg to at most 3 mg DHA per g dry weight of the composition.

### Subject

The present use is for allergic subjects. Allergic subjects not only include subjects that have been diagnosed to have an allergy, but also subjects that have an increased risk of developing an allergy such as infants of parents having an allergy. The present use is specifically intended for allergic infants and/or allergic toddlers. Infants have an age of 0-12 months, toddlers have an age of 12-36 months, even more preferably for infants. Thus in one instance according to the present invention, the allergic subject is an allergic infant and/or toddler.

### Application and compositions

The present use is for the treatment or prevention, preferably the prevention of infections in subjects with an allergy.

The composition according to the present use is preferably enterally administered, more preferably orally. The present composition is preferably a nutritional formula, preferably an infant formula. The present composition can advantageously be applied as a complete nutrition for infants. The present composition preferably comprises lipid, protein, and carbohydrate and is preferably administered in liquid form. The present invention includes dry compositions, e.g. powders, which are accompanied with instructions as to admix said dry compositions, in particular nutritional formula, with a suitable liquid, e.g. water.

In a preferred instance in the composition for the use according to the present invention, the soluble indigestible fiber comprises fructooligosaccharide and the lactic acid bacterium is *Bifidobacterium breve.*

In one instance in the composition for use according to the present invention the soluble indigestible fiber comprises a mixture of short chain fructooligosaccharide with an average degree of polymerization from 2 to 6 and long chain fructooligosaccharide with an average degree of polymerization of at least 7, and the weight ratio short chain fructooligosaccharide : long chain fructooligosaccharide is at least 1, preferably the weight ratio scFOS : lcFOS between 2 and 12, even more preferably between 3 and 10, most preferably the weight ratio scFOS : lcFOS is about 9.

In one instance the composition for use according to the present invention comprises i) a protein source, ii) a prebiotic and iii) a probiotic, wherein i) the protein source consists of free amino acids and is present in between 10 and 20 wt% based on the dry weight of the total composition, ii) the prebiotic comprises a mixture of short chain fructooligosaccharide with an average degree of polymerisation from 2 to 6 and long chain fructooligosaccharide with an average degree of polymerisation of at least 7, and the weight ratio short chain fructooligosaccharide : long chain fructooligosaccharide is at least 1, and iii) the probiotic comprises at least one lactic acid bacterium selected from the group consisting of *Bifidobacterium breve, Bifidobacterium longum, Bifidobacterium infantis, Bifidobacterium lactis* and *Lactobacillus rhamnosus.*

In a preferred instance the composition for use according to the present invention is a nutritional composition comprising an allergen free protein source, essentially consisting of free amino acids, and Bifidobacteria, preferably Bifidobacterium breve, and a source of non digestible carbohydrates comprising fructooligosaccharides with an average DP of 2-60.

In yet a further preferred instance the composition for use according to the present invention is a nutritional composition, preferably an infant formula, comprising a protein source, a fat source, soluble indigestible fiber, and live lactic acid bacteria, wherein the protein source essentially consist of free amino acids and provides from 7 to 20% of the total calories of the nutritional composition, the fat source comprises at least arachidonic acid (AA) and docosahexaenoic acid (DHA), energy percent, the soluble indigestible fiber comprises fructooligosaccharides with an average DP of 2-60 in a concentration from 15 to 75 mg per g dry weight of the nutritional composition and the live lactic acid bacteria are selected from the group consisting of *Bifidobacteria* and *Lactobacillus rhamnosus,* preferably selected from the group consisting of *Bifidobacterium breve* and *Lactobacillus rhamnosus LGG,* preferably the lactic acid bacteria comprise *Bifidobacterium breve.*

In a preferred instance in the composition for use according to the present invention the protein source provides from 10 to 20% of the total calories of the composition, the concentration of soluble indigestible fiber is from 15 to 75 mg per g dry weight of the composition and the concentration of lactic acid bacteria, preferably *Bifidobacterium breve,* is 2.0x10⁸ and 2.0x10¹⁰ CFU lactic acid bacteria, preferably *Bifidobacterium breve,* per gram soluble indigestible fiber, more preferably between 1.0x10⁹ and 1.0x10¹⁰ CFU lactic acid bacteria, preferably *Bifidobacterium breve,* per gram soluble indigestible fiber, most preferably between 1.0x10⁹ and 5.0x10⁹ CFU lactic acid bacteria, preferably *Bifidobacterium breve,* per gram soluble indigestible fiber. Preferably the soluble indigestible fiber is a mixture of short chain fructooligosaccharide with an average degree of polymerization from 2 to 6 and long chain fructooligosaccharide with an average degree of polymerization of at least 7, and the weight ratio short chain fructooligosaccharide : long chain fructooligosaccharide is at least 1. Preferably the weight ratio scFOS : lcFOS between 2 and 12, even more preferably between 3 and 10, most preferably the weight ratio scFOS : lcFOS is about 9. Preferably the composition further comprises fat providing 30 to 50% of the total calories of the composition, and the composition comprises DHA or ARA or both in a concentration of at least 0.35 mg per gram dry weight of the composition, preferably from at least 0.4 mg to at most 10 mg ARA per g dry weight of the composition and/or from at least 0.4 mg to at most 10 mg DHA per g dry weight of the composition, preferably from at least 0.5 mg to at most 6 mg ARA per g dry weight of the composition and/or from at least 0.5 mg to at most 6 mg DHA per g dry weight of the composition, more preferably from at least 0.6 mg to at most 3 mg ARA per g dry weight of the composition and/or from at least 0.6 mg to at most 3 mg DHA per g dry weight of the composition.

### EXAMPLES

### Example 1. Clinical study showing the anti-inflammatory effects of a synbiotic prebiotic fiber mix with Bifidobacterium breve in a population of allergic patients.

Pre- and probiotics (synbiotics) were investigated for the potential beneficial effects on human health. This study describes the functional effects of an amino-acid based formula (AAF) with synbiotics in infants with cow's milk allergy (CMA).

### Methods

In a prospective, randomized, double-blind controlled study, full term infants with IgE and/or non-IgE mediated CMA randomly received a commercially available AAF (NEO; n=56) or an AAF with synbiotics (NEO-SYN; n=54) for 16 weeks. Primarily, infant growth and tolerance of the formula was monitored. Secondarily, dermatological (including severity of atopic manifestations by SCORAD) and respiratory allergic characteristics and stool characteristics were either recorded in subject diaries and/or evaluated by a physician. The NEO-SYN group were exclusively fed with a commercially available AAF supplemented with a milk protein free *Bifidobacterium breve* and a prebiotic fiber mix comprising short chain fructooligosaccharides (scFOS, with an average degree of polymerization below 6) and lcFOS (with an average degree of polymerisation above 7) in a weight ratio scFOS : lcFOS of approximately 9:1 in a concentration of about 45 mg scFOS + lcFOS per gram dry weight of the composition. The B. *breve* strain used was the commercially available strain M-16V of Morinaga. *B. breve* was used in a concentration of 1.9x10⁹ colony forming units (CFU) per gram prebiotic fiber.

### Results

Average age of infants at inclusion was 4.58±2.45 months. Overall NEO-SYN and NEO were equally well tolerated and both supported normal growth. Both formulas reduced allergic symptoms, and no significant differences between the groups were observed; The NEO-SYN group was reported to have less subjects suffering from infections (p=0.008) and less subjects receiving medication for functional gastrointestinal (GI) disorders (p=0.029) when compared with the NEO group. In addition the NEO-SYN group had a lower number of infants with antibiotics usage (p=0.049), especially amoxicillin (p=0.004), compared with the NEO group. The results are summarised in table 1.

**Table 1: Reported infections and antibiotic use in window of 16 weeks**

| | NEO | NEO-SYN | *p*-value |
|---|---|---|---|
| Infections (reported) | 17.9% | 1.9% | 0.008 |
| Antibiotic use | | | |
| - overall | 33.9% | 16.7% | 0.049 |
| - amoxicillin | 32.1% | 9.3% | 0.004 |

### Conclusion

This study shows that an AAF with synbiotics is equally well tolerated, supports normal growth and has similar efficacy to manage CMA symptoms compared to an AAF without synbiotics. Addition of synbiotics improves resistance against infections and reduces specific medication usage in infants receiving AAF.

### Example 2. Composition for use according to the invention

| | UNIT | per 100g | per 100ml* |
|---|---|---|---|
| **energy:** | kcal | 483 | 67 |
| **Protein** (see table 2): | g | 13 | 1.8 |
| % of total energy | | 10.8 | 10.8 |
| **Carbohydrate:** | g | 52.5 | 7.3 |
| Sugars | g | 4.7 | 0.65 |
| % of total energy | | 43.5 | 43.5 |
| **Fat:** | g | 24.5 | 3.4 |
| % of total energy | | 45.7 | 45.7 |
| Saturates | g | 8.9 | 1.2 |
| Monounsaturates g | | 9.6 | 1.3 |
| Polyunsaturates | g | 4.8 | 0.67 |
| DHA | mg | 110 | 15 |
| ARA | mg | 110 | 15 |
| **Prebiotic fibre**: | g | 4.9 | 0.68 |
| Ratio scFOS/lcFOS about 9:1 | | | |
| **Lactic acid bacteria**: B.breve M-16V - 1.9x10⁹ (CFU) per gram prebiotic fiber | | | |

| | | | |
|---|---|---|---|
| *14.7 g powder is dissolved in 100 ml water | | | |

**Table 2: Composition of protein source**

| COMPONENT | UNIT | Per 100 g composition |
|---|---|---|
| **Amino Acids** | | |
| L-Alanine | g | 0,6 |
| L-Arginine | g | 1,0 |
| L-Aspartic acid | g | 1,0 |
| L-Cystine | g | 0,4 |
| L-Glutamine | g | 1,3 |
| Glycine | g | 0,9 |
| L-Histidine | g | 0,6 |
| L-Isoleucine | g | 0,9 |
| L-Leucine | g | 1,6 |
| L-Lysine | g | 1,1 |
| L-Methionine | g | 0,2 |
| L-Phenylalanine | g | 0,7 |
| L-Proline | g | 1,1 |
| L-Serine | g | 0,7 |
| L-Threonine | g | 0,8 |
| L-Tryptophan | g | 0,3 |
| L-Tyrosine | g | 0,7 |
| L-Valine | g | 1,0 |
| L-Carnitine | g | 0,01 |

## Claims

1. Composition comprising i) a protein source, ii) a prebiotic and iii) a probiotic for use in the treatment or prevention of infection in an allergic subject, wherein
i. the protein source consists of free amino acids,
ii. the prebiotic comprises a mixture of
a. short chain fructooligosaccharide with an average degree of polymerisation from 2 to 6, and
b. long chain fructooligosaccharide with an average degree of polymerisation of at least 7, and
the weight ratio short chain fructooligosaccharide : long chain fructooligosaccharide is at least 1,
iii. the probiotic is *Bifidobacterium breve.*

2. The composition for use according to claim 1, wherein the protein source provides from 7 to 20% of the total calories of the composition.

3. The composition for use according to any one of claims 1-2, wherein the content of the protein source is between 10 and 20 wt% free amino acids based on dry weight of the total composition.

4. The composition for use according to any one of claims 1-3, wherein the composition further comprises DHA or ARA or both.

5. The composition for use according to any one of claims 1-4, wherein the protein source provides from 10 to 20% of the total calories of the composition, the concentration of soluble indigestible fiber is from 15 to 75 mg per g dry weight of the composition and the concentration of lactic acid bacteria is between 2.0x10⁸ and 2.0x10¹⁰ CFU lactic acid bacteria per gram soluble indigestible fiber.

6. The composition for use according to claim 5, wherein the composition further comprises fat providing 30 to 50% of the total calories of the composition, and the composition comprises DHA or ARA or both in a concentration of at least 0.35 mg per gram dry weight of the composition.

7. The composition for use according to any one of claims 1 - 6, wherein the composition does not comprise uronic acid oligosaccharide.

8. The composition for use according to any one of claims 1 - 7, wherein the allergic subject is an infant.

9. The composition for use according to any one of claims 1 - 8, wherein the composition is an infant formula.

## Patentansprüche

1. Zusammensetzung umfassend i) eine Proteinquelle, ii) ein Präbiotikum und iii) ein Probiotikum zur Verwendung in der Behandlung oder Vermeidung einer Infektion bei einer allergischen Person, wobei
i. die Proteinquelle aus freien Aminosäuren besteht,
ii. das Präbiotikum eine Mischung umfasst aus
a. kurzkettigem Fructooligosaccharid mit einem durchschnittlichen Polymerisationsgrad von 2 bis 6, und
b. langkettigem Fructooligosaccharid mit einem durchschnittlichen Polymerisationsgrad von wenigstens 7, und
wobei das Gewichtsverhältnis von kurzkettigem Fructooligosaccharid : langkettigem Fructooligosaccharid wenigstens 1 ist,
iii. das Probiotikum *Bifidobacterium breve* ist.

2. Zusammensetzung zur Verwendung nach Anspruch 1, wobei die Proteinquelle 7 bis 20% der gesamten Kalorien der Zusammensetzung bereitstellt.

3. Zusammensetzung zur Verwendung nach einem der Ansprüche 1 bis 2, wobei der Gehalt der Proteinquelle zwischen 10 und 20 Gew.-% freie Aminosäuren, basierend auf einem Trockengewicht der gesamten Zusammensetzung, ist.

4. Zusammensetzung zur Verwendung nach einem der Ansprüche 1 bis 3, wobei die Zusammensetzung ferner DHA oder ARA oder beides umfasst.

5. Zusammensetzung zur Verwendung nach einem der Ansprüche 1 bis 4, wobei die Proteinquelle 10 bis 20% der gesamten Kalorien der Zusammensetzung bereitstellt, die Konzentration der löslichen unverdaulichen Faser von 15 bis 75 mg pro Gramm Trockengewicht der Zusammensetzung ist und die Konzentration an Milchsäurebakterien zwischen 2,0 x 10⁸ und 2,0 x 10¹⁰ CFU-Milchsäurebakterien pro Gramm löslicher unverdaulicher Faser liegt.

6. Zusammensetzung zur Verwendung nach Anspruch 5, wobei die Zusammensetzung ferner Fett umfasst, bereitstellend 30 bis 50% der gesamten Kalorien der Zusammensetzung, und wobei die Zusammensetzung DHA oder ARA oder beides in einer Konzentration von wenigstens 0,35 mg pro Gramm Trockengewicht der Zusammensetzung umfasst.

7. Zusammensetzung zur Verwendung nach einem der Ansprüche 1 bis 6, wobei die Zusammensetzung kein Uronsäureoligosaccharid umfasst.

8. Zusammensetzung zur Verwendung nach einem der Ansprüche 1 bis 7, wobei die allergische Person ein Kleinkind ist.

9. Zusammensetzung zur Verwendung nach einem der Ansprüche 1 bis 8, wobei die Zusammensetzung eine Kleinkindmilchnahrung ist.

## Revendications

1. Composition comprenant i) une source de protéines, ii) un prébiotique et iii) un probiotique à utiliser dans le traitement ou la prévention d'une infection chez un sujet allergène, dans laquelle
i. la source de protéines se compose d'acides aminés libres,
ii. le prébiotique comprend un mélange de
a. fructo-oligosaccharides à chaîne courte avec un degré moyen de polymérisation de 2 à 6, et
b. fructo-oligosaccharides à longue chaîne avec un degré moyen de polymérisation d'au moins 7, et
le rapport en poids fructo-oligosaccharides à chaîne courte : fructo-oligosaccharides à chaîne longue est d'au moins 1,
iii. le probiotique est *Bifidobacterium breve.*

2. Composition à utiliser selon la revendication 1, dans laquelle la source de protéines fournit de 7 à 20 % des calories totales de la composition.

3. Composition à utiliser selon l'une quelconque des revendications 1 à 2, dans laquelle la teneur de la source de protéines est comprise entre 10 et 20 % en poids d'acides aminés libres sur la base du poids sec de la composition totale.

4. Composition à utiliser selon l'une quelconque des revendications 1 à 3, dans laquelle la composition comprend en outre de l'ADH ou de l'ARA, ou les deux.

5. Composition à utiliser selon l'une quelconque des revendications 1 à 4, dans laquelle la source de protéines fournit de 10 à 20 % des calories totales de la composition, la concentration de fibres non digestibles solubles est de 15 à 75 mg par gramme de poids sec de la composition, et la concentration de bactéries d'acide lactique est comprise entre 2,0x10⁸ et 2,0x10¹⁰ CFU de bactéries d'acide lactique par gramme de fibres non digestibles solubles.

6. Composition à utiliser selon la revendication 5, dans laquelle la composition comprend en outre de la matière grasse fournissant 30 à 50 % des calories totales de la composition, et la composition comprend de l'ADH ou de l'ARA ou les deux à une concentration d'au moins 0,35 mg par gramme de poids sec de la composition.

7. Composition à utiliser selon l'une quelconque des revendications 1 à 6, dans laquelle la composition ne comprend pas d'oligosaccharide d'acide uronique.

8. Composition à utiliser selon l'une quelconque des revendications 1 à 7, dans laquelle le sujet allergène est un nourrisson.

9. Composition à utiliser selon l'une quelconque des revendications 1 à 8, dans laquelle la composition est une formule pour nourrissons.
